(19) Europäisches Patentamt

European Patent Office

Office européen des brevets

(11) **EP 2 609 913 A1**

(12) **EUROPEAN PATENT APPLICATION**

(43) Date of publication:
**03.07.2013 Bulletin 2013/27**

(51) Int Cl.:
*A61K 9/70* *(2006.01)*    *A61F 13/02* *(2006.01)*

(21) Application number: **12199165.7**

(22) Date of filing: **21.12.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **26.12.2011 JP 2011283713**

(71) Applicant: **Nitto Denko Corporation**
**Osaka 567-8680 (JP)**

(72) Inventors:
• **Aoyagi, Kazuhiro**
  **Ibaraki-shi, Osaka 567-8680 (JP)**
• **Iwao, Yoshihiro**
  **Ibaraki-shi, Osaka 567-8680 (JP)**
• **Matsuoka, Kensuke**
  **Ibaraki-shi, Osaka 567-8680 (JP)**
• **Tanaka, Tomoya**
  **Ibaraki-shi, Osaka 567-8680 (JP)**

(74) Representative: **Jackson, Martin Peter**
**J A Kemp**
**14 South Square**
**Gray's Inn**
**London WC1R 5JJ (GB)**

(54) **Patch preparation**

(57) The present invention relates to a patch preparation having a central part and a peripheral part surrounding the central part, the patch preparation comprising: a patch preparation main body having a support and an adhesive layer which contains a drug and is formed on the support; and a release liner laminated on the adhesive layer, wherein the peripheral part has a predetermined area, and a peel force between the adhesive layer and the release liner in the predetermined area is greater than a peel force between the adhesive layer and a release liner in the central part.

**FIG. 1**

**Description**

TECHNICAL FIELD OF THE INVENTION

The present invention relates to a patch preparation wherein an adhesive layer containing a drug is formed on one entire surface of a support, and a release liner is laminated on the adhesive layer to cover the entire exposed surface of the adhesive layer.

BACKGROUND OF THE INVENTION

As a preparation for administering a drug to the body for the treatment or prophylaxis of a disease, for example, transdermal absorption type preparations can be mentioned, which can avoid drug metabolism due to the first-pass through the liver and various side effects, and can sustainably administer a drug for a long time. Among those, a patch preparation containing a drug in an adhesive has been developed, since an dispensing work is easy and the dosage can be strictly controlled.

In general, patch preparations have a preparation main body consisting of a support made of a woven fabric, a plastic film and the like and an adhesive layer containing a drug laminated on the support, and a release liner laminated on the adhesive layer of the preparation main body. They are provided in a package made of a packaging material such as a resin film and the like.

In recent years, a thicker adhesive layer is often employed to contain a large amount of a drug in an adhesive layer. As other characteristic of recent patch preparations, a soft adhesive layer tends to be employed such as an adhesive layer containing a large amount of a liquid component therein and the like, in an attempt to improve a soft feeling on adhesion to the skin, or reduce skin irritation due to the detachment of stratum corneum during peeling. In such patch preparations, since the adhesive layer is thick and soft, an end portion of the patch preparation highly frequently contacts the inner surface of the package when the patch preparation is taken out from the package. As a result, terminal edge lifting, that is, a phenomenon of lifting, from the release liner, of the edge of an adhesive layer on an end portion of the preparation main body occurs. As a result, the surface of the exposed adhesive layer attaches to the inner surface of the package or the hand of workers, which markedly degrades taking-out performance and handleability. In such patch preparations, moreover, the above-mentioned end edge lifting is easily developed in a punching out step during the production and in a packaging step, thus sometimes lowering the yield.

Examples of the documents dealing with such problems include JP-A-10-310108 and JP-A-6-199659. JP-A-10-310108 proposes setting the peel force between a drug-containing adhesive layer and a separator to fall within a particular range, and forming a concave/convex on an exposed surface of a support, an exposed surface of a separator and at least one inner surface of the packaging material to be in contact with a drug-containing adhesive sheet. The "separator" means the same as the "release liner". However, since such drug-containing adhesive sheet has a high peel force over the entire surface, a large force is necessary for detaching the drug-containing adhesive sheet from the separator when in use, which in turn places a high burden on the workers and sometimes degrades handleability.

JP-A-6-199659 proposes a transdermal treatment apparatus having no adhesive layer in the central part but having a pressure-sensitive adhesive layer involved in the adhesion to the skin only on the periphery (what is called a reservoir type transdermal preparation), wherein a particularly preferable embodiment proposed therein is a transdermal treatment apparatus comprised of a drug release layer comprising a backing layer for a drug, a drug reservoir tank, and a porous material treated with a nonionic surfactant, a pressure sensitive adhesive layer formed on the periphery of a drug release surface of the drug release layer, and a release liner layer. In such transdermal treatment apparatus, an adhesive force is present only on the periphery and the central part does not have an adhesive force. Therefore, a small force is necessary to detach the release liner layer. To ensure a sufficient adhesive force to the skin, the actual area of the apparatus (hereinafter sometimes to be referred to as "actual area") becomes larger than the effective area of the apparatus which is determined by the releaseability of the drug. Consequently, the QOL (Quality of Life) of patients may be degraded as evidenced by lower sense of use (feeling of adhesion and the like) of the user (patients) and the like. Moreover, since the actual area of the apparatus is large, a large packaging material is necessary for packing the apparatus, which problematically increases the material cost.

BRIEF SUMMARY OF THE INVENTION

In view of the above-mentioned situation, the problem to be solved by the present invention is to provide a patch preparation having an actual area of the preparation not larger than the effective area of the preparation necessary for drug releaseability, which resists easy end edge lifting when taking the preparation out from the package, and when in use, and permits easy detachment of the preparation main body from the release liner.

In addition, the problem is to provide a patch preparation which resists easy end edge lifting in a punching out step and/or a packaging step, as well as when taking the preparation out from the package, and which permits easy detachment of the preparation main body from the release liner when in use.

The present inventors have conducted intensive studies in an attempt to solve the aforementioned problems and found that the above-mentioned problem can be effectively solved by setting the peel force between the adhesive layer and the release liner in at least one part of the peripheral part surrounding the central part of a patch preparation to be greater than that of the peel force between the adhesive layer and the release liner in the central part, which resulted in the completion of the present invention.

Accordingly, the present invention provides the following.

[1] A patch preparation having a central part and a peripheral part surrounding the central part, the patch preparation comprising: a patch preparation main body having a support and an adhesive layer which contains a drug and is formed on the support; and a release liner laminated on the adhesive layer, wherein the peripheral part has a predetermined area, and a peel force between the adhesive layer and the release liner in the predetermined area is greater than a peel force between the adhesive layer and a release liner in the central part.

[2] The patch preparation according to [1], wherein the patch preparation has a substantially rectangular or a substantially square planar shape.

[3] The patch preparation according to [2], wherein the peripheral part has four corner areas, and the predetermined area is at least one of the four corner areas.

[4] The patch preparation according to [2], wherein the peripheral part has four comer areas and the predetermined area is one pair of two countering corner areas out of the four corner areas.

[5] The patch preparation according to [2], wherein the peripheral part has four corner areas, and the predetermined area is the four corner areas.

[6] The patch preparation according to [2], wherein the peripheral part has four sides, and the predetermined area is an area along one side out of the four sides.

[7] The patch preparation according to [2], wherein the peripheral part has four sides, and the predetermined area is two areas along each one pair of the two countering sides out of the four sides.

[8] The patch preparation according to [1] or [2], wherein the predetermined area is an entire area of the peripheral area.

[9] The patch preparation according to any one of [1] to [8], wherein the peel force of the predetermined area is 5 to 500 mN/2mm.

[10] The preparation according to any one of [1] to [9], wherein the peel force of the predetermined area is greater than the peel force between the adhesive layer and a release liner in the central part by not less than 3 mN/2mm.

[11] The patch preparation according to any one of [1] to [10], wherein the peripheral area has a width of 0.5 to 10 mm.

[12] The patch preparation according to any one of [1] to [11], wherein the adhesive layer has a thickness of 10 to 500 μm

[13] The patch preparation according to any one of [1] to [12], wherein the adhesive layer comprises an organic liquid component.

[14] The patch preparation according to any one of [1] to [13], wherein the release liner covers the entire exposed surface of the adhesive layer of the patch preparation main body.

[15] The patch preparation according to [14], wherein an end portion of the release liner protrudes from the end portion of the patch preparation main body.

[16] The patch preparation according to any one of [1] to [15], wherein the release liner has a continuous cutting line.

In the patch preparation of the present invention, the peel force between the adhesive layer and the release liner in at least one part of the peripheral part (that is, a predetermined area of the peripheral part) is greater than that in the central part. Therefore, end edge lifting can be prevented when taking the patch preparation out from the package, thereby reducing the possibility of decreased handleability of the patch preparation during and after taking the preparation out from the package.

In addition, since end edge lifting in a punching out step during production of a patch preparation and/or a packaging step of the patch preparation can be prevented, a decrease of the yield can be avoided.

During use of the patch preparation, moreover, the adhesive layer can be easily detached from the release liner by simply picking the release liner with a hand after detaching the adhesive layer on the peripheral part from the release liner, since the peel force between the adhesive layer and the release liner is small in the central part, which in turn increases the adhesion workability of the patch preparation.

In an embodiment with a continuous cutting line for division formed in a release liner, the patch preparation can be easily detached from the release liner by simply picking the release liner with a hand from the continuous cutting line, for the same reason as above.

Furthermore, in the patch preparation of the present invention, since an adhesive layer containing a drug is formed on one entire surface of a support, the actual area of the preparation is the effective area of the preparation, which avoids the preparation from becoming larger than necessary.

BRIEF DESCRIPTION OF THE DRAWING

Fig. 1(A) and Fig. 1(B) are a plane view and a sectional view showing one embodiment of the patch preparation of the present invention.
Fig. 2 is a partial view of the inside of the package housing the patch preparation of Fig. 1.
Fig. 3 schematically shows the manner of taking out a patch preparation from the package of Fig. 2.
Fig. 4(A) - Fig. 4(E) schematically show a preferable embodiment of the patch preparation of the present invention having a substantially rectangular planar shape.

In the Figures, 1 is a support, 2 is an adhesive layer, 3 is a preparation main body, 4 is a release liner, 5 is a patch preparation, 5A is a central part, 5B is the peripheral part, 6 is a packing film, 7 is a cut-out portion, 8 is a high peel force part, 10 is a package, and 41 is a continuous cutting line.

DETAILED DESCRIPTION OF THE INVENTION

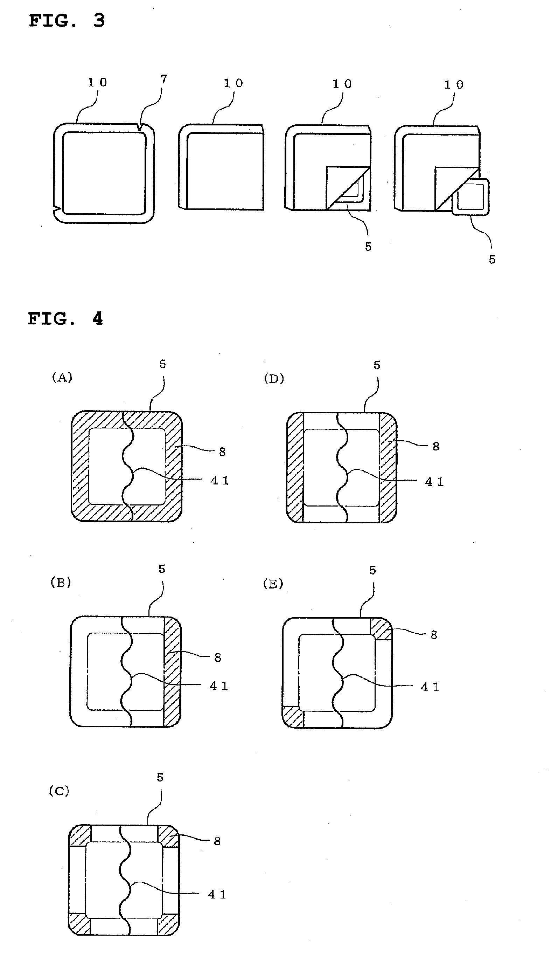

While the present invention is explained in the following by referring to preferable embodiments, the detailed explanation and particular examples thereof are merely shown for exemplification purposes, and do not limit the present invention, and application thereof or use thereof.

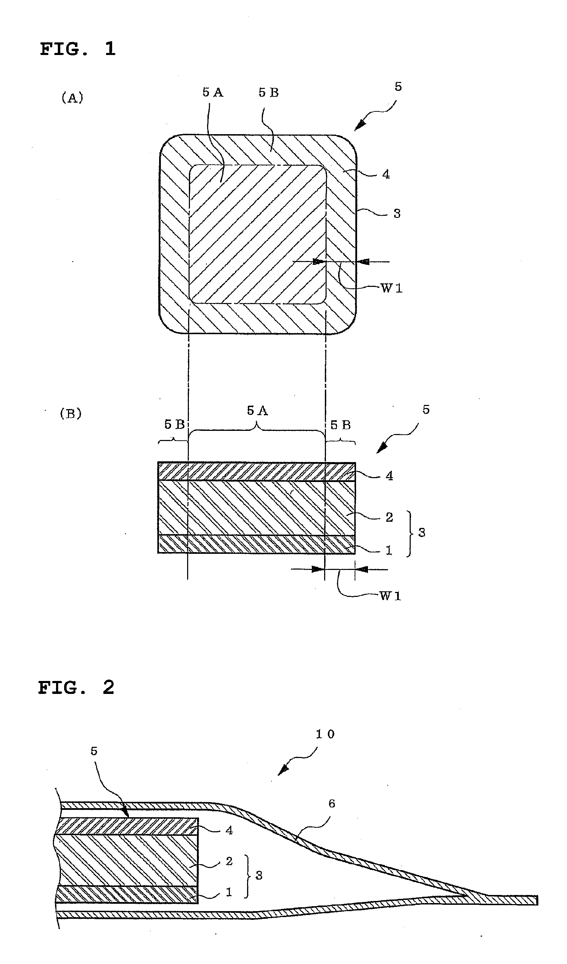

Fig. 1 shows schematic drawings of one embodiment of the patch preparation of the present invention, wherein Fig. 1(A) is a plane view and Fig. 1(B) is a sectional view.
The patch preparation of the present invention comprises, as shown in the patch preparation 5 of one embodiment, a laminate of a preparation main body 3 comprised of an adhesive layer 2 containing a drug, which is formed on one entire surface of support 1, and a release liner 4 laminated on the adhesive layer 2 to cover the entire exposed surface of the adhesive layer 2 of the preparation main body 3. The patch preparation of the present invention has a central part 5A, and a peripheral part 5B surrounding the central part, wherein the peel force between the adhesive layer 2 and the release liner 4 in at least one part of the peripheral part 5B, that is, a predetermined area of the peripheral part 5B, is greater than that in the central part 5A.
In the patch preparation of the present invention, "the central part" and "the peripheral part" are, as shown in Fig. 1 (A), based on the central part and the peripheral part surrounding same in a planar view of the patch preparation. In Fig. 1(A), for ease of explanation, the boundary between the central part 5A and the peripheral part 5B is shown with a two-dot chain line, and the central part 5A and the peripheral part 5B are each hatched to visually distinguish the central part 5A from the peripheral part 5B. However, actual patch preparations may not have such boundary lines and hatching.
Generally, a patch preparation after production is housed in a package to suppress deterioration, protect the patch preparation from the outside impact and the like and shipped, and when in use, taken out from the package and used. The same applies to the patch preparation of the present invention, and it is also housed in a package after production. Fig. 2 partially shows the inside of the package 10 housing the patch preparation 5 of the present invention. A patch preparation 5 is housed in a package 10 wherein peripheral end portions of the packaging films 6 facing each other over the patch preparation 5 are sealed. Fig. 3 schematically shows the manner of taking out a patch preparation 5 from the patch preparation package 10 of Fig. 2. The package 10 in Fig. 3 has two cut-out portions 7 from which to open the packaging film, and the packaging film 6 is cut from the cut-out portion 7 to open the package 10 and the patch preparation 5 is taken out, as shown therein.
In the patch preparation 5 of the present invention, since the peel force between the adhesive layer 2 and the release liner 4 in a predetermined area (e.g., hatched area 8 in the below-mentioned Fig. 4(A) - Fig. 4(E)) in the peripheral part 5B is greater than that of the peel force between the adhesive layer 2 and the release liner 4 in the central part 5A, In an operation to take out the patch preparation 5 from the package 10 as shown in Fig. 3, even when the end portion of the patch preparation 5 contacts the inner surface of the package 10, end edge lifting, i.e., a phenomenon of an end portion of the adhesive layer 2 of the preparation main body 3 lifting from the release liner 4, can be prevented. Therefore, the possibility of degraded workability of taking out the patch preparation 5 from the package 10 and degraded handleability of the patch preparation 5 after taking out can be reduced. In addition, during the use of the patch preparation, the adhesive layer 2 can be easily detached from the release liner 4 by simply picking the release liner 4 with a hand after detaching the adhesive layer 2 in the peripheral part 5B from the release liner 4, since the peel force between the adhesive layer 2 and the release liner 4 in the central part 5A is small, which in turn also improves the adhesion workability.

In the patch preparation 5 of the present invention, the peel force between the adhesive layer 2 and the release liner 4 in the peripheral part 5B may be greater in one part of the peripheral part 5B than that of the peel force between the adhesive layer 2 and the release liner 4 in the central part 5A, or greater in the entirety of the peripheral part 5B. That is, a predetermined area of the peripheral part 5B wherein the peel force between the adhesive layer 2 and the release liner 4 is greater than that in the central part 5A (hereinafter to be also referred to as "high peel force area") may be partially formed in the peripheral part 5B or formed in the entire area of the peripheral part 5B. The high peel force area preferably occupies at least 2% or above of the planar area of the whole peripheral part 5B. When the high peel force area is partially formed in the peripheral part 5B, the peel force between the adhesive layer 2 and the release liner 4 in the areas other than the high peel force area of the peripheral part 5B is generally the same as that in the central part 5A.

In the patch preparation of the present invention, the size of the patch preparation is specified by the planar area of the preparation main body 3 wherein the adhesive layer 2 containing a drug is formed on one entire surface of the support 1. The planar area of the preparation main body 3 is appropriately set according to the kind of the drug to be contained in the adhesive layer 2, the adhesive to be used for the adhesive layer 2 and the like, and is not particularly limited. Generally, it is 1 - 200 cm$^2$, preferably 2 - 100 cm$^2$. In the patch preparation 5 shown in Fig. 1(A) and Fig. 1(B), the release liner 4 covering the entire exposed surface of the adhesive layer 2 of the preparation main body 3 has the same planar shape as the preparation main body 3. However, the release liner 4 may have a larger planar size than the preparation main body 3. In this case, the end portion of the release liner 4 may protrude from the end portion of the preparation main body 3 over the complete outer circumference of the preparation main body 3, or may protrude in a part of the outer circumference of the preparation main body 3. When the planar size of the release liner is larger than the preparation main body, the part of the release liner protruding from the outer circumference of the preparation main body can be held by hand, which facilitates detachment of the adhesive layer (preparation main body) from the release liner during use of the patch preparation.

While the protrusion length of the end portion of the release liner (length of protrusion from the end portion of the preparation main body) is not particularly limited, when it is too long, the workability of taking the patch preparation out from the package may be degraded. When the package size is increased to prevent degradation of the workability, the package cost increases. Therefore, the protrusion length of the end portion of the release liner (length of protrusion from the end portion of the preparation main body) is preferably about 2 -10 mm.

In the patch preparation of the present invention, the peel force between the adhesive layer 2 and the release liner 4 in the central part 5A is basically the same in the entire central part 5A, and is not particularly limited. Even when the peripheral part 5B has a high peel force area, when the peel force between the adhesive layer 2 and the release liner 4 in the central part 5A is too small (particularly when the release liner has a cutting line for separation, and the patch preparation is folded by the outside stress and the like), easy detachment of the release liner 4 from the preparation main body 3 in the central part 5A is feared. Therefore, the peel force between the adhesive layer 2 and the release liner 4 in the central part 5A is preferably 2 mN/2 mm or above, more preferably 3 mN/2 mm or above. In consideration of the detach workability of the preparation main body 3 from the release liner 4 during use of the patch preparation, the peel force is preferably 60 mN/2 mm or less, more preferably 20 mN/2 mm or less.

The peripheral part 5B in the patch preparation of the present invention is, as shown in Fig. 1(A) and Fig. 1(B), a band-like portion having a predetermined width along the side surface of patch preparation (preparation main body) ("band-like" here is the shape in the planar view of Fig. 1 (A)). While the width thereof (W1 in Fig. 1) is not particularly limited, it is preferably not less than 0.5 mm, more preferably not less than 1 mm, to more certainly prevent the end edge lifting of the patch preparation. When the width of the peripheral part 5B is too large, the adhesive layer 2 in the peripheral part 5B may not be detached from the release liner 4 with ease during use of the patch preparation. Thus, it is preferably not more than 10 mm, more preferably not more than 8 mm.

The peel force of the high peel force area in the peripheral part 5B is preferably 5 mN/2 mm or above, more preferably 7 mN/2 mm or above, still more preferably 10 mN/2 mm or above, particularly preferably 25 mN/2 mm or above. When the peel force in the high peel force area is too high, a large power is necessary to detach the preparation main body from the release liner during use of the patch preparation, possibly imposing a burden on the users. Therefore, the peel force of the high peel force area is preferably 500 mN/2 mm or less. To more stably achieve the effect of the invention, the peel force of the high peel force area is preferably greater by 3 mN/2 mm or above, more preferably 5 mN/2 mm or above, than the peel force between the adhesive layer 2 and the release liner 4 in the central part 5A.

In the patch preparation of the present invention, the thickness of the adhesive layer 2 is appropriately set according to the kind of a drug to be contained in the adhesive layer 2, the kind of an adhesive to be used for the adhesive layer 2 and the like, and is not particularly limited. Generally, it is 10 - 500 $\mu$m, preferably 20 - 400 $\mu$m. When it is less than 10 $\mu$m, the adhesiveness to the skin may not be sufficient. When it exceeds 500 $\mu$m, the improvement of the adhesiveness may hit the ceiling, and the material cost may become high. The thickness of the adhesive layer 2 may be substantially the same over the entire surface of the preparation main body, or the thickness of the adhesive layer in the peripheral part may be smaller than the thickness of the adhesive layer in the central part 5A. The "substantially the same" here means that the thickness variation relative to a predetermined thickness (object value) is within the range of ±25% of the predetermined thickness. By setting the thickness of the adhesive layer 2 in the peripheral part 5B to be smaller than the thickness of the adhesive layer 2 in the central part 5A, the taking-out performance of the patch preparation from the package is further improved.

The adhesive layer 2 is a layer-like structure containing a drug and a polymer and shows adhesiveness at ambient temperature (25°C).

The polymer to be contained in the adhesive layer is not particularly limited, and examples thereof include acrylic polymers including (meth)acrylic acid ester polymer; rubber polymers such as styrene-isoprene-styrene block copolymer, styrene-butadiene-styrene block copolymer, polyisoprene, polyisobutylene, polybutadiene and the like; silicone polymers such as silicone rubber (preferably silicone rubber based on dimethylsiloxane, silicone rubber based on diphenylsiloxane) and the like; vinylalkylether polymers such as poly(vinylmethylether), poly(vinylethylether), poly(vinylisobutylether) and the like; vinylester polymers such as ethylene-vinyl acetate copolymer and the like; ester polymer comprised of a carboxylic acid component such as dimethylterephthalate, dimethylisophthalate, dimethylphthalate and the like and a polyvalent alcohol component such as ethyleneglycol and the like, and the like.

As an acrylic polymer, preferred is one obtained by copolymerization of (meth)acrylic acid alkyl ester as a main component and a functional monomer. That is, a copolymer comprising 50 - 99 wt% (preferably 60 - 95 wt%) of a monomer component consisting of (meth)acrylic acid alkyl ester, wherein the rest of the monomer component is a functional monomer, is preferable. The main component here means a monomer component contained in a proportion of not less than 50 wt% of the total weight of the monomer components constituting the copolymer.

The (meth)acrylic acid alkyl ester (hereinafter to be also referred to as the main component monomer) is generally that wherein the alkyl group is a straight chain or branched chain alkyl group having 4 -13 carbon atoms. Examples therof include linear alkylmono(meth)acrylate such as n-butyl(meth)acrylate, pentyl(meth)acrylate, hexyl (meth)acrylate, heptyl(meth)acrylate, octyl(meth)acrylate, nonyl(meth)acrylate, decyl(meth)acrylate, undecyl(meth)acrylate, dodecyl(meth)acrylate, tridecyl acrylate and the like; branched chain alkylmono(meth)acrylate such as sec-butyl (meth)acrylate, tert-butyl(meth)acrylate, 2-methylhexyl(meth)acrylate, 2-ethylhexyl(meth)acrylate, 2-propylhexyl(meth) acrylate, 2-methylheptyl(meth)acrylate, 2-ethylheptyl(meth)acrylate, 2-propylheptyl(meth)acrylate and the like. One or more kinds of these are used.

The functional monomer has at least one unsaturated double bond, which is involved in a copolymerization reaction, in a molecule and a functional group on the side chain. Examples thereof include carboxyl group-containing monomer such as (meth)acrylic acid, itaconic acid, maleic acid, maleic anhydride and the like, hydroxyl group-containing monomer such as hydroxyethyl(meth)acrylate, hydroxypropyl(meth)acrylate and the like; sulfo group-containing monomer such as styrene sulfonic acid, allyl sulfonic acid, sulfopropyl(meth)acrylate, (meth)acryloyloxy naphthalenesulfonate, 2-acrylamide-2-methylpropanesulfonic acid and the like; amino group-containing monomer such as 2-(methylamino) ethyl acrylate, 1-(methylamino)ethyl acryliate, 2-(dimethylamino)ethyl (meth)acrylate, 1-(tert-butylamino)ethyl(meth)acrylate, 2-(tert-butylamino)ethyl(meth)acrylate and the like; amide group-containing monomer such as (meth)acrylamide, N,N-dimethyl(meth)acrylamide, N-methylol(meth)acrylamide, N-methylolpropane(meth)acrylamide, N-vinylacetamide and the like; alkoxyl group-containing monomer such as methoxyethyl(meth)acryliate, ethoxyethyl(meth)acrylate, methoxyethylene glycol(meth)acrylate, methoxydiethylene glycol(meth)acrylate, methoxypolyethylene glycol(meth)acrylate, methoxypolypropylene glycol(meth)acrylate, tetrahydrofurfryl(meth)acrylate and the like.

One or more kinds of such functional monomers can be used. Of those, a carboxyl group-containing monomer is preferable, and (meth)acrylic acid is particularly preferable from the aspects of pressure-sensitive adhesiveness of an adhesive layer, cohesiveness, releaseability of a drug contained in the adhesive layer and the like.

As the acrylic polymer, one obtained by further copolymerizing the above-mentioned copolymer of (meth)acrylic acid alkyl ester (main component monomer) and a functional monomer with other monomer can also be used.

Examples of such other monomer include (meth)acrylonitrile, vinyl acetate, vinyl propionate, N-vinyl-2-pyrrolidone, vinylpyridine(2-vinylpyridine, 4-vinylpyridine), N-vinylpiperidone, vinylpyrimidine(2-vinylpyrimidine, 4-vinylpyrimidine), vinylpiperazine, N-vinylpyrrole, N-vinylimidazole, N-vinyl-ε-caprolactam, vinyloxazole and the like. One or more kinds of these can be used.

The amount of such other monomer to be used is generally preferably about 0 - 40 wt%, more preferably about 10 - 30 wt%, relative to the total weight of the (meth)acrylic acid alkyl ester (main component monomer) and the functional monomer.

As the acrylic polymer, a terpolymer of 2-ethylhexyl acrylate as (meth)acrylic acid alkyl ester, acrylic acid and N-vinyl-2-pyrrolidone is preferable, and a copolymer obtained by copolymerizing 2-ethylhexyl acrylate, acrylic acid and N-vinyl-2-pyrrolidone at a weight ratio of 40 - 99.9:0.1 - 10:0 - 50 is more preferable, since good adhesiveness to the human skin can be achieved, and adhesion and detachment can be easily repeated.

As the rubber polymer, one containing at least one kind selected from polyisobutylene, polyisoprene and styrene-diene-styrene block copolymer (styrene-butadiene-styrene block copolymer (SBS), styrene-isoprene-styrene block copolymer (SIS) etc.) as the main component is preferable. Since high drug stability, and necessary adhesive force and cohesion strength can be simultaneously achieved, a rubber polymer mixture of high molecular weight-polyisobutylene having a viscosity average molecular weight of 500,000 - 2,100,000, and low molecular weight-polyisobutylene having a viscosity average molecular weight of 10,000 - 200,000 at a weight ratio of 95:5 - 5:95 is particularly preferable.

The viscosity average molecular weight here is obtained by calculating the Staudinger index ($J_0$) according to the Schulz-Blaschke equation from the flow time of capillary of the Ubbelohde's viscometer at 20°C, and applying the $J_0$ value to the following equations.

[formula 1]

$$J_0 = \eta_{sp}/\{c(1+0.31\eta_{sp})\} \text{ (Schulz-Blaschke equation)}$$

$$\eta_{sp} = t/t_0 - 1$$

t: flow time of solution (according to Hagenbach-couette correction)
$t_0$: flow time of solvent (according to Hagenbach-couette correction)
c: concentration of solution (g/cm$^3$)

$$J_0 = 3.06 \times 10^{-2} \, Mv^{0.65}$$

Mv: viscosity average molecular weight

When a rubber polymer is used, it is preferable to further add a tackifier to the adhesive layer 2 to impart sufficient adhesiveness at ambient temperature to the adhesive layer 2. The tackifier is not particularly limited, and those known in the technical field may be appropriately selected and used. Examples thereof include petroleum resin (e.g., aromatic petroleum resin, aliphatic petroleum resin and the like), terpene resin, rosin resin, coumarone indene resin, styrene resin (e.g., styrene resin, poly($\alpha$-methylstyrene) and the like), hydrogenated petroleum resin (e.g., alicyclic saturated hydrocarbon resin and the like) and the like. Of these, an alicyclic saturated hydrocarbon resin is preferable, since the preservation stability of the drug becomes fine. One or more kinds of tackifiers can be used in combination, and the amount of the tackifier is generally 33 - 300 wt%, preferably 50 - 200 wt%, relative to the total weight of the rubber polymer.

The drug contained in the adhesive layer 2 is not particularly limited, and a transdermally absorbable drug that can be administered to mammals such as human and the like through the skin is preferable. Specific examples of such drug include general anesthetics, hypnotic sedatives, antiepileptic drugs, antipyretic analgesic antiphlogistic drugs, anti-vertiginous drugs, psychoneurotic drugs, central nervous drugs, topical anesthetics, skeleton muscle relaxants, autonomic drugs, antispasmodic drugs, anti-parkinsonian drugs, anti-histamine drugs, cardiac stimulants, drugs for arrhythmia, diuretic, hypotensive drug, vasoconstrictor, coronary vasodilator, peripheral vasodilators, arteriosclerosis drugs, drugs for circulatory organ, anapnoics, antitussive expectorant, hormone drugs, external drugs for purulent diseases, analgesic-antipruritic-styptic-antiinflammatory drugs, drugs for parasitic skin diseases, hemostatic drugs, gout treatment drugs, drugs for diabetes, anti-malignant tumor agents, antibiotic, chemical therapy agents, narcotic, quit smoking aids and the like.

The drug is present in the adhesive layer in an amount sufficient (namely, effective amount) to provide desired results (for example, desired therapeutic effect), in the treatment of disease, condition or disorder. To be specific, the effective amount of the drug means, for example, an amount of the drug that provides a concentration of the drug in blood lower than a toxic level and sufficient to provide a selected effect over a predetermined time. Such amount can be easily determined by those of ordinary skill in the art. The amount of the drug in the adhesive layer is not particularly limited as long as it provides the effect of the transdermally absorbable drug and does not impair adhesion property of the adhesive. For example, the amount to be contained is 0.1 - 60 wt%, preferably 0.5 - 40 wt%, relative to the whole adhesive layer. When the amount is less than 0.1 wt%, the treatment effect may be insufficient, and when it exceeds 60 wt%, the content of an adhesive constituting the adhesive layer becomes small, and sufficient skin adhesiveness may not be achieved, which may be economically disadvantageous.

When desired, for example, for adjustment of adhesiveness, acceleration of transdermal absorption of a drug and the like, the adhesive layer 2 may contain an organic liquid component. The organic liquid component is an organic compound which is liquid at room temperature (25°C) and plasticizes an adhesive layer. Examples thereof include glycols such as ethylene glycol, diethylene glycol, triethylene glycol, propylene glycol, polyethylene glycol, polypropylene glycol and the like; fats and oils such as olive oil, castor oil, squalene, lanolin and the like; organic solvent such as ethyl acetate, ethyl alcohol, dimethyldecyl sulfoxide, methyloctyl sulfoxide, dimethyl sulfoxide, dimethylformamide, dimethylacetamide, dodecylpyrrolidone, isosorbitol and the like; liquid surfactant; plasticizers such as octyldodecanol, diisopropyladipate, phthalic acid ester, diethylsebacate, triethyl citrate, acetylcitric acid tributyl and the like; hydrocarbons such as liquid paraffin and the like; ethoxylated stearyl alcohol; fatty acid ester; glycerol acid ester and the like.

A fatty acid ester comprised of a higher fatty acid having a carbon number of 12 - 16, (more preferably 12 - 14) and a lower monovalent alcohol having a carbon number of preferably 1 - 4 is preferable. Examples of the higher fatty acid having a carbon number of 12 -16 include lauric acid, myristic acid, palmitic acid and the like, and examples of the lower monovalent alcohol having a carbon number of 1 - 4 include methyl alcohol, ethyl alcohol, propyl alcohol, isopropyl alcohol and the like. In addition, as the glycerol acid ester, glycerol middle chain (carbon number 8 - 12) fatty acid ester is preferable, and may be any one of monoglyceride, diglyceride and triglyceride, or a mixture of two or more kinds thereof.

One or more kinds of the organic liquid component can be used. The content of the organic liquid component in the adhesive layer 2 is preferably 5 - 60 wt%, more preferably 10 - 50 wt%, of the whole adhesive layer. When the content is less than 5 wt%, the adhesive layer may not be plasticized sufficiently, a good soft feeling may not be obtained, or skin irritation may not be decreased sufficiently. Conversely, when it exceeds 60 wt%, the organic liquid component cannot be maintained in the adhesive even by the cohesion strength possessed by the adhesive, it causes blooming on the surface of the adhesive layer, thus resulting in too weak adhesive force, which in turn may cause falling off of the preparation from the skin surface during use.

In addition, the adhesive layer may be applied to physical crosslinking by irradiation such as ultraviolet irradiation, electron beam irradiation and the like, or chemical crosslinking treatment using an isocyanate compound such as trifunctional isocyanate and the like and various crosslinking agents such as organic peroxide, organic metal salt, metal alcoholate, metal chelate compound, multifunctional compound (multifunctional external crosslinking agent, or multifunctional internal crosslinkable monomer such as diacrylate, dimethacrylate and the like) and the like to give a crosslinked adhesive layer. The crosslinked adhesive layer containing an organic liquid component is preferable since it has appropriate skin adhesiveness due to its gel state, and cohesive property that does not leave an adhesive residue on detachment.

While the support 1 is not particularly limited in the patch preparation of the present invention, it is specifically, for example, a single film such as polyester (e.g., poly(ethylene terephthalate) (PET) etc.), nylon, saran(trade name), polyvinyl chloride, polyethylene, polypropylene, ethylene-vinyl acetate copolymer, polytetrafluoroethylene, ionomer resin and the like, metal foil, or a laminate film of two or more kinds of films selected therefrom and the like. To improve adhesiveness (anchor property) of a support to an adhesive layer, the support may be a laminate film of a non-porous film comprised of the above-mentioned material and a porous film mentioned below, and an adhesive layer is preferably formed on the side of the porous film.

The porous film is not particularly limited as long as it improves the anchor property to an adhesive layer and, for example, paper, woven fabric, non-woven fabric (e.g., polyester (e.g., poly(ethylene terephthalate) (PET) and the like) non-woven fabric and the like), the above-mentioned film (single film of polyester (e.g., poly(ethylene terephthalate) (PET) etc.), nylon, saran (trade name), polyvinyl chloride, polyethylene, polypropylene, ethylene-vinyl acetate copolymer, polytetrafluoroethylene, ionomer resin and the like, metal foil, and a laminate film by laminating one or more kinds of these and the like) and the like can be mentioned. Particularly, paper, woven fabric and non-woven fabric (e.g., polyester non-woven fabric, poly(ethylene terephthalate) non-woven fabric and the like) are preferable to afford flexibility of the support. When a porous film, for example, woven fabric or non-woven fabric is used, the weight thereof is preferably 5 - 30 g/m$^2$ to improve the anchor property.

The laminate film as a support is produced by a known production method of a laminate film such as dry lamination method, wet lamination method, extrusion lamination method, hot melt lamination method, coextrusion lamination method and the like.

While the thickness of the support is not particularly limited, it is preferably 2 - 200 $\mu$m, more preferably 10 - 50 $\mu$m. When it is less than 2 $\mu$m, the preparation main body 3 loses strength, the handling property during application, and adhesion workability and the like tend to decrease. When it exceeds 200 $\mu$m, an unpleasant feeling (a feeling of stiffness) is produced to often degrade the followability.

The release liner in the patch preparation of the present invention is not particularly limited, and a plastic film of polyester (e.g., poly(ethylene terephthalate) and the like), polyvinyl chloride, polyvinylidene chloride and the like, paper such as quality paper, glassine and the like, or a laminate film wherein two or more kinds selected therefrom are laminated and the like, each of which is peel treated by applying a silicone release agent, a fluorine release agent and the like, are used. The thickness of the release liner is generally 10 $\mu$m - 200 $\mu$m, preferably 50 $\mu$m 150 $\mu$m.

In the patch preparation of the present invention, a method of setting the peel force between the adhesive layer and the release liner in a predetermined area of the peripheral part to be greater than that in the central part (that is, a method of forming a high peel force area of the peripheral part) includes (I) a method of changing the surface property of the release liner between a predetermined area of the peripheral part and the central part, and (II) a method of changing the surface property of the adhesive layer between a predetermined area of the peripheral part and the central part.

As (I) a method of changing the surface property of the release liner between a predetermined area of the peripheral part and the central part, the following method can be specifically mentioned.

(1) In the production step of the release liner, a UV curable silicone release agent solution is uniformly applied to the entire surface of a substrate film (e.g., PET film), the UV irradiation amount is changed between a part of the release liner corresponding to the central part of a patch preparation to be obtained later and an area corresponding to a predetermined area of the peripheral part, whereby the cure extent of the silicone release agent is changed.

(2) In the production step of the release liner, a thermoset silicone release agent solution is uniformly applied to the entire surface of a substrate film (e.g., PET film), the heating temperature or heating time is changed between a part of the release liner corresponding to the central part of a patch preparation to be obtained later and an area corresponding to a predetermined area of the peripheral part, whereby the cure extent of the silicone release agent is changed.

(3) In the release liner, a part of the release liner corresponding to the central part of a patch preparation to be obtained later is coated with a release agent having a low peel force, and an area corresponding to a predetermined area of the peripheral part is coated with a release agent having a high peel force or is not coated with a release agent.

(4) In the release liner, a release agent of an area corresponding to a predetermined area of the peripheral part of the patch preparation to be obtained later is partly or entirely removed by abrasion with a metal piece and the like.

(5) In the release liner, an area corresponding to a predetermined area of the peripheral part of the patch preparation to be obtained later is pressed with a heated die and the like to make the release agent partly or entirely fall off.

(6) A release liner having a high peel force and a release liner having a low peel force are prepared, and the release liner having a low peel force is laminated and fixed only on an area of the release liner having a high peel force, which corresponds to the central part of the patch preparation to be obtained later.

(7) In the release liner, an area corresponding to a predetermined area of the peripheral part of the patch preparation to be obtained later is thermally or mechanically treated to make the release agent of the release liner partly or entirely fall off.

As (II) a method of changing the surface property of the adhesive layer between a predetermined area of the peripheral part and the central part, the following method can be specifically mentioned.

(1) A part to be the central part of a patch preparation to be obtained later is coated with an adhesive layer having a low peel force, and an area to be a predetermined area of the peripheral part is coated with an adhesive layer having a high peel force.

(2) A release liner of a patch preparation is detached, the concentration of the crosslinking agent to be applied to the adhesive layer is changed between an area to be the central part of a patch preparation and an area to be a predetermined area of the peripheral part, whereby the degree of crosslinking of the adhesive layer is changed between the central part and the predetermined area of the peripheral part.

(3) An adhesive layer having a low peel force is formed on the entire surface of the patch preparation, and an adhesive layer having a high peel force is laminated on the adhesive layer of the patch preparation except an area to be the central part.

(4) An adhesive layer having a high peel force is formed on the entire surface of the patch preparation, and an appropriate amount of a powder (e.g., inorganic particles such as white talc and the like, organic particles such as crospovidone (polyvinylpyrrolidone) and the like, etc.) is carried only on an area to be the central part of the adhesive layer of the patch preparation to lower the peel force only of the central part.

The planar shape of the patch preparation of the present invention is not particularly limited. While the patch preparation 5 shown in Fig. 1(A) and Fig. 1(B) has a substantially square planar shape, it may be, for example, a planar shape such as a substantially rectancular, ellipse, circular shape and the like. Here, each of the "a substantially square shape" and "a substantially rectancular shape" means a shape in which a corner area thereof does not form a right angle but forms a rounded shape. In view of the handling property of the patch preparation, improved yield of the punching out step for the production of the patch preparation and the like, the planar shape of the patch preparation is preferably a substantially square or a substantially rectangular shape.

In addition, the release liner 4 may have a continuous cutting line at a predetermined position. When a release liner 4 has a continuous cutting line, a patch preparation can be more easily detached from a release liner by picking the portion. That is, the patch preparation can be easily detached from the release liner by simply picking the release liner with a hand from the continuous cutting line.

Fig. 4(A) - 4(E) show preferable embodiments of the patch preparation of the present invention wherein the planar shape is a substantial square. The Figures show planar views on the release liner side, wherein the hatched parts are the predetermined areas (high peel force areas) 8 formed in the peripheral part 5B and showing a greater peel force between the adhesive layer and the release liner than that in the central part 5A. In all embodiments, the release liner 4 has a continuous cutting line 41.

Fig. 4(A) is an embodiment wherein the entire area of the peripheral part 5B is a high peel force area (first embodiment), Fig. 4(B) is an embodiment wherein an area along one of the four sides of the peripheral part 5B is a high peel force area (second embodiment), Fig. 4(C) is an embodiment wherein four corner areas of the peripheral part 5B are high peel force areas (third embodiment), Fig. 4(D) is an embodiment wherein the areas along a pair of countering two sides of the four sides of the peripheral part 5B are high peel force areas (fourth embodiment), and Fig. 4(E) is an embodiment wherein the areas along a pair of countering two corner areas from the four corner areas of the peripheral part 5B are high peel force areas (fifth embodiment). In all embodiments, the areas other than the high peel force area 8 in the peripheral part 5B show the same peel force between the adhesive layer and the release liner as in the central part 5A.

As is clear from the below-mentioned Examples, those five embodiments show good taking-out performance from the package and easy detachment of the release liner during adhesion operation.

When the planar shape of a patch preparation is a substantially square shape or asubstantially rectangular shape, end edge lifting tends to occur particularly at a corner area. In any of the first to fifth embodiments, therefore, at least two corner areas in the peripheral part are high peel force areas.

Depending on the position of the cut-out portion 7 formed in the package 10, the position of opening of the package is determined (see Fig. 3), and the direction of the patch preparation to be taken out from the package is limited. The end edge lifting can be more certainly prevented by the embodiment of Fig. 4(E) wherein the countering two corner areas are high peel force areas, and the embodiment of Fig. 4(B) wherein one side of the four sides (including two corner areas) is a high peel force area, rather than the embodiment wherein only one from the four corner areas is a high peel force area.

Moreover, the embodiment of Fig. 4(C) wherein the four corner areas are high peel force areas, and the embodiment of Fig. 4(D) wherein a pair of countering two sides (including four corner areas) are high peel force areas can certainly prevent end edge lifting without being influenced by the direction of taking out the patch preparation from the package, and the embodiment of Fig. 4(A) wherein the entire area of the peripheral part is a high peel force area can more certainly prevent end edge lifting. However, in the embodiment of Fig. 4(A), the easiness of the detachment of the release liner somewhat decreases.

A patch preparation having a predetermined planar shape can be generally obtained by producing a laminate having a laminate constitution of a substrate film for release liner/adhesive layer/substrate film for support, and subjecting the laminate to a punching out processing.

In the production of the patch preparation of the present invention, a laminate patch preparation having a greater peel force between the adhesive layer and the release liner in a predetermined area of the peripheral part than that in the central part is formed in a laminate, which prevents end edge lifting in a laminate punching out step. In addition, end edge lifting due to the contact of the patch preparation with the inner surface of the package during an operation to house each patch preparation, obtained by punching out, in each package can also be prevented. Therefore, an effect of avoiding a lower yield can be afforded.

Particularly, when a patch preparation has a substantially square or a substantially rectanglar shape, at least four corner areas of the peripheral part are preferably high peel force areas so as to prevent end edge lifting in a punching out step and/or a packaging step. Thus, the embodiment of Fig. 4(A) wherein the entire area of the peripheral part is a high peel force area, the embodiment of Fig. 4(C) wherein four corner areas are high peel force areas and the embodiment of Fig. 4(D) wherein areas along a pair of countering two sides out of the four sides are high peel force areas are preferable.

<Measurement method of peel force between adhesive layer of preparation main body and release liner>

The measurement method of the peel force between the preparation main body and the release liner of the patch preparation of the present invention is as described below.

A band-like test piece having width 2 mm, any length is prepared from the patch preparation. That is, a patch preparation is cut in the thickness direction thereof (laminating direction of the laminate) and, where necessary, aid paper is adhered to give a band-like test piece (width 2 mm x length 50 mm). At one end of the test piece in the longitudinal direction, about 5 mm is peeled from the release liner end portion and folded back at an angle of 180°, and the aid paper (width 5 mm) is adhered to extend the length. Using a tensile tester, one end of the aid paper is nipped by the upper clasp, the release liner and the plate for backing (e.g., Bakelite (trade name) plate) are nipped by the lower clasp, the adhesive sheet is continuously peeled off in the 180 degree direction at 300 mm/min under an atmosphere of 23°C$\pm$2°C, 50$\pm$10%RH, and the peel force is measured.

**Examples**

The present invention is explained in detail in the following by referring to Examples and Comparative Examples, which are not to be construed as limitative.

Example 1

<Preparation of composition for forming adhesive layer>

Polyisobutylene 1 having a high molecular weight (viscosity average molecular weight 820,000), polyisobutylene 2 having a low molecular weight (viscosity average molecular weight 55,000), a tackifier (alicyclic saturated hydrocarbon resin, softening point 100°C (ring and ball method)), an organic liquid component (isopropyl palmitate), and a drug (tulobuterol) were mixed at a weight ratio of 26:28:26:15:5 in the presence of toluene to give a composition for forming an adhesive layer.

<Preparation of adhesive sheet>

The above-mentioned composition was applied to an easy peeling surface of a poly(ethylene terephthalate) (hereinafter to be abbreviated as PET) release liner (thickness 75 $\mu$m) such that the thickness of an adhesive layer after drying was 200 $\mu$m, and dried in a drying oven (100°C, 5 min) to give a release liner having an adhesive layer. The surface having the adhesive layer was laminated on a PET non-woven fabric surface of a laminate (total thickness: about 35 $\mu$m) of a 2 $\mu$m-thick PET film and the PET non-woven fabric (fabric weight 12 g/m$^2$), which is a support, by pressure-bonding to give an adhesive sheet.

<Replacing and punching out of release liner>

The release agent on an area (width 2 mm) of a PET release liner (thickness 75 $\mu$m) different from the above, that corresponds to the peripheral part of a patch preparation (with square planar shape) to be produced later, was completely removed by rubbing with a metal piece, and a wavy line-like cutting part was formed on the release liner such that the middle points of the countering two sides of the patch preparation to be produced later were connected.

A release liner adhered to the above-mentioned adhesive sheet was detached, and an easy peeling surface of the above-mentioned PET release liner wherein the release agent on the area corresponding to the peripheral part of the patch preparation to be produced later had been completely removed was adhered to the surface where an adhesive layer of the adhesive sheet was formed. The adhesive sheet was punched out using Thompson blade (60 mm x 60 mm square) to give the patch preparation of Example 1 having an appearance of Fig. 4(A). The peripheral area was 4.6 cm$^2$, and the proportion (area ratio) of the high peel force area in the peripheral part was 100%.

Example 2

<Preparation of composition for forming an adhesive layer>

A composition similar to that in Example 1 was used.

<Preparation of adhesive sheet>

An adhesive sheet was obtained in the same manner as in Example 1.

<replacing punching out of release liner>

The release agent of an area along one of the four sides in the area (width 2 mm) of a different PET release liner (thickness 75 $\mu$m), that corresponds to the peripheral part of a patch preparation (with square planar shape) to be produced later, was removed by rubbing with a metal piece, and a wavy line-like cutting part was formed on the release liner such that the middle points of the countering two sides of the patch preparation to be produced later were connected. A release liner adhered to the above-mentioned adhesive sheet was detached, and an easy peeling surface of the above-mentioned PET release liner wherein the release agent of the area along one of the four sides in the area corresponding to the peripheral part of the patch preparation to be produced later had been removed was adhered to the surface where an adhesive layer of the adhesive sheet was formed. The adhesive sheet was punched out using Thompson blade (60 mm x 60 mm square) to give the patch preparation of Example 2 having an appearance of Fig. 4 (B). The proportion (area ratio) of the high peel force area in the peripheral part was 25%.

Example 3

<Preparation of composition for forming an adhesive layer>

A composition similar to that in Example 1 was used.

<Preparation of adhesive sheet>

An adhesive sheet was obtained in the same manner as in Example 1.

<replacing and punching out of release liner>

The release agent of four corner areas in the area (width 2 mm) of a different PET release liner (thickness 75 $\mu$m), that corresponds to the peripheral part of a patch preparation (with square planar shape) to be produced later, was removed by rubbing with a metal piece, and a wavy line-like cutting part was formed on the release liner such that the middle points of the countering two sides of the patch preparation to be produced later were connected. A release liner adhered to the above-mentioned adhesive sheet was detached, and an easy peeling surface of the above-mentioned PET release liner wherein the release agent of the four corner areas in the area corresponding to the peripheral part of the patch preparation to be produced later had been removed was adhered to the surface where an adhesive layer of the adhesive sheet was formed. The adhesive sheet was punched out using Thompson blade (60 mm x 60 mm square) to give the patch preparation of Example 3 having an appearance of Fig. 4(C). The proportion (area ratio) of the high peel force area in the peripheral part was 10%.

Example 4

<Preparation of composition for forming an adhesive layer>

Polymer amounts of polyisobutylene 1 (viscosity average molecular weight 820,000), low molecular weight polyisobutylene 2 (viscosity average molecular weight 55,000), a tackifier (alicyclic saturated hydrocarbon resin, a softening point 100°C (ring and ball method)), an organic liquid component 1 (isopropyl palmitate), an organic liquid component 2 (diethylene glycol monododecylether), and a drug (tulobuterol) were mixed at a weight ratio of 26:23:26:19:1: 5 in the presence of toluene to give a composition for forming an adhesive layer.

<Production of adhesive sheet>

The above-mentioned composition was applied to an easy peeling surface of a PET release liner (thickness 75 $\mu$m) such that the thickness of the adhesive layer after drying was 200 $\mu$m, dried by a dryer (100°C, 5 min) to give a release liner with an adhesive layer. The adhesive surface (surface on which an adhesive layer is formed) was press-adhered to a PET non-woven fabric surface of a laminate (total thickness: about 35 $\mu$m) of a 2 $\mu$m-thick PET film and the PET non-woven fabric (fabric weight 12 g/m$^2$), which is a support, to give an adhesive sheet.

<Replacing and punching out of release liner>

The release agent of the entire area (width 2 mm) of a different PET release liner (thickness 75 $\mu$m), that corresponds to the peripheral part of a patch preparation (with square planar shape) to be produced later, was removed by rubbing with a metal piece, and a wavy line-like cutting part was formed on the release liner such that the middle points of the countering two sides of the patch preparation to be produced later were connected. A release liner adhered to the above-mentioned adhesive sheet was detached, and an easy peeling surface of the above-mentioned PET release liner wherein the release agent of the entire area corresponding to the peripheral part of the patch preparation to be produced later had been removed was adhered to the surface where an adhesive layer of the adhesive sheet was formed. The adhesive sheet was punched out using Thompson blade (60 mm x 60 mm square) to give the patch preparation of Example 4 having an appearance of Fig. 4(A). The proportion (area ratio) of the high peel force area in the peripheral part was 100%.

Example 5

<Preparation of composition for forming an adhesive layer>

A composition similar to that in Example 4 was used.

<Preparation of adhesive sheet>

An adhesive sheet was obtained in the same manner as in Example 4.

<replacing and punching out of release liner>

The release agent of an area along each side of a pair of countering two sides out from the four sides of the area (width 2 mm) of a different PET release liner (thickness 75 $\mu$m), that corresponds to the peripheral part of a patch preparation (with square planar shape) to be produced later, was removed by rubbing with a metal piece, and a wavy line-like cutting part was formed on the release liner such that the middle points of the countering two sides of the patch preparation to be produced later were connected. A release liner adhered to the above-mentioned adhesive sheet was detached, and an easy peeling surface of the above-mentioned PET release liner wherein the release agent of the area along each side of the pair of countering two sides out from the four sides of the area corresponding to the peripheral part of the patch preparation to be produced later had been removed was adhered to the surface where an adhesive layer of the adhesive sheet was formed. The adhesive sheet was punched out using Thompson blade (60 mm x 60 mm square) to give the patch preparation of Example 5 having an appearance of Fig. 4(D). The proportion (area ratio) of the high peel force area in the peripheral part was 51%.

Example 6

A composition similar to that in Example 4 was used.

<Preparation of adhesive sheet>

An adhesive sheet was obtained in the same manner as in Example 4.

<replacing and punching out of release liner>

The release agent of two corner areas located on the diagonal line in the area (width 2 mm) of a different PET release liner (thickness 75 $\mu$m), that corresponds to the peripheral part of a patch preparation (with square planar shape) to be produced later, was removed by rubbing with a metal piece, and a wavy line-like cutting part was formed on the release liner such that the middle points of the countering two sides of the patch preparation to be produced later were connected. A release liner adhered to the above-mentioned adhesive sheet was detached, and an easy peeling surface of the above-mentioned PET release liner wherein the release agent of two corner areas located on the diagonal line in the area corresponding to the peripheral part of the patch preparation to be produced later had been removed was adhered to the surface where an adhesive layer of the adhesive sheet was formed. The adhesive sheet was punched out using Thompson blade (60 mm x 60 mm square) to give the patch preparation of Example 6 having an appearance of Fig. 4(E). The proportion (area ratio) of the high peel force area in the peripheral part was 5%.

Example 7

<Preparation of composition for forming adhesive layer>

Polyisobutylene 1 having a high molecular weight (viscosity average molecular weight 820,000), polyisobutylene 2 having a low molecular weight (viscosity average molecular weight 55,000), a tackifier (alicyclic saturated hydrocarbon resin, softening point 100°C (ring and ball method)), an organic liquid component 1 (isopropyl palmitate), an organic liquid component 2 (tetraethyleneglycol monododecylether), and a drug (tulobuterol) were mixed at a weight ratio of 26:23:26:18:2:5 in the presence of toluene to give a composition for forming an adhesive layer.

<Preparation of adhesive sheet>

The above-mentioned composition was applied to an easy peeling surface of a PET release liner (thickness 75 $\mu$m) such that the thickness of an adhesive layer after drying was 200 $\mu$m, and dried in a drying oven (100°C, 5 min) to give a release liner having an adhesive layer. The surface having the adhesive layer was laminated on a PET non-woven fabric surface of a laminate (total thickness: about 35 $\mu$m) of a 2 $\mu$m-thick PET film and the PET non-woven fabric (fabric weight 12 g/m$^2$), which is a support, by pressure-bonding to give an adhesive sheet.

<Replacing and punching out of release liner>

The release agent of the entire area (width 2 mm) of a different PET release liner (thickness 75 $\mu$m), that corresponds to the peripheral part of a patch preparation (with square planar shape) to be produced later, was removed by rubbing with a metal piece, and a wavy line-like cutting part was formed on the release liner such that the middle points of the countering two sides of the patch preparation to be produced later were connected. A release liner adhered to the above-mentioned adhesive sheet was detached, and an easy peeling surface of the above-mentioned PET release liner wherein the release agent of the entire area corresponding to the peripheral part of the patch preparation to be produced later had been removed was adhered to the surface where an adhesive layer of the adhesive sheet was formed. The adhesive sheet was punched out using Thompson blade (60 mm x 60 mm square) to give the patch preparation of Example 7 having an appearance of Fig. 4(A). The proportion (area ratio) of the high peel force area in the peripheral part was 100%.

Example 8

<Preparation of composition for forming adhesive layer>

Under an inert gas atmosphere, 2-ethylhexyl acrylate (95 parts by weight), acrylic acid (5 parts by weight) and benzoyl peroxide (0.2 part by weight) were subjected to solution polymerization in ethyl acetate at 60°C to give an acrylic adhesive solution.
The acrylic adhesive, an organic liquid component (isopropyl palmitate), and a drug (isosorbide dinitrate) were mixed at a weight ratio of 43:40:17 in the presence of ethyl acetate to give a composition for forming an adhesive layer.

<Preparation of adhesive sheet>

The above-mentioned composition was applied to an easy peeling surface of a release liner (thickness 75 $\mu$m) made of PET such that the thickness of the adhesive layer after drying was 200 $\mu$m, and dried in a drying oven (100°C, 3 min) to give a release liner comprising an adhesive layer. The adhesive surface (surface on which an adhesive layer is formed) was press-adhered to a PET non-woven fabric surface of a laminate (total thickness: about 35 $\mu$m) of a 2 $\mu$m-thick PET film and the PET non-woven fabric (fabric weight 12 g/m$^2$), which is a support, to give an adhesive sheet.

<Coating of crosslinking agent solution, promotion of crosslinking reaction and punching out>

The release liner of the adhesive sheet was detached, an ethyl acetate solution of CORONATEHL (manufactured by NIPPON POLYURETHANE INDUSTRY Co., Ltd.) as a crosslinking agent was applied to an area of an adhesive sheet, which corresponds to an area along each side of a pair of countering two sides out from the four sides of the area (width 2 mm) of a different PET release liner (thickness 75 $\mu$m), that corresponds to the peripheral part of a patch preparation (with square planar shape) to be produced later, at 0.05 parts by weight per 100 parts by weight of the acrylic adhesive solid content. An ethyl acetate solution of CORONATEHL as a crosslinking agent was applied to an area along each side of a pair of countering two sides of the area (width 2 mm) to the peripheral part of a patch preparation to be produced later, and the central part, at 0.15 parts by weight per 100 parts by weight of the acrylic adhesive solid content, and they were dried by a dryer (100°C, 3 min). An easy peeling surface of PET release liner (thickness 75 $\mu$m) wherein a wavy line-like cutting part was formed such that the middle points of the countering two sides of the patch preparation to be produced later were connected was adhered to the surface where the adhesive layer was formed to give an adhesive sheet. The adhesive sheet was tightly sealed in a package material (outer size 600 mm×250 mm, inner size 580 mm×230 mm) with an outer layer made of a 12 $\mu$m-thick PET film and an inner layer made of a 30 $\mu$m-thick polyacrylonitrile based resin film, left standing in a thermostat at 70°C for 48 hr to promote a crosslinking reaction of the adhesive layer. The adhesive sheet was punched out using Thompson blade (60 mm x 60 mm square) to give the patch preparation of Example 8 having an appearance of Fig. 4(D). The proportion (area ratio) of the high peel force area in the peripheral part was 51%.

Comparative Example 1

<Preparation of composition for forming adhesive layer>

Polyisobutylene 1 having a high molecular weight (viscosity average molecular weight 820,000), polyisobutylene 2 having a low molecular weight (viscosity average molecular weight 55,000), a tackifier (alicyclic saturated hydrocarbon resin, softening point 100°C (ring and ball method)), an organic liquid component (isopropyl palmitate), and a drug (tulobuterol) were mixed at a weight ratio of 26:33:26:10:5 in the presence of toluene to give a composition for forming an adhesive layer.

<Production of adhesive sheet>

The above-mentioned composition was applied to an easy peeling surface of a PET release liner (thickness 75 $\mu$m) such that the thickness of the adhesive layer after drying was 200 $\mu$m, dried by a dryer (100°C, 5 min) to give a release liner with an adhesive layer. The adhesive surface (surface on which an adhesive layer is formed) was press-adhered to a PET non-woven fabric surface of a laminate (total thickness: about 35 $\mu$m) of a 2 $\mu$m-thick PET film and the PET non-woven fabric (fabric weight 12 g/m$^2$), which is a support, to give an adhesive sheet.

<Replacing and punching out of release liner>

A wavy line-like cutting part was formed on a different PET release liner (thickness 75 $\mu$m, without peel treatment) such that the middle points of the countering two sides of the patch preparation (with square planar shape) to be produced later were connected. A release liner adhered to the adhesive sheet was detached and the release liner having the cutting part was adhered to the surface where an adhesive layer of the adhesive sheet was formed. The adhesive sheet was punched out using Thompson blade (60 mm x 60 mm square) to give the patch preparation of Comparative Example 1.

Comparative Example 2

<Preparation of composition for forming an adhesive layer>

A composition similar to that in Example 7 was used.

<Preparation of adhesive sheet>

An adhesive sheet was obtained in the same manner as in Example 7.

<replacing and punching out of release liner>

A wavy line-like cutting part was formed on a different PET release liner (thickness 75 $\mu$m) such that the middle points of the countering two sides of the patch preparation (with square planar shape) to be produced later were connected. A release liner adhered to the adhesive sheet was detached and an easy peeling surface of the release liner having the cutting part was adhered to the surface where an adhesive layer of the adhesive sheet was formed. The adhesive sheet was punched out using Thompson blade (60 mm x 60 mm square) to give the patch preparation of Comparative Example 20.
The patch preparations of Examples 1 - 8 and Comparative Examples 1 - 2 were evaluated for the following evaluation items.

Experimental Example 1

<Measurement method of peel force between patch preparation and release liner>

A band-like test piece having width 2 mm, any length was prepared from the central part and the peripheral part (area treated to have high peel force area) of a patch preparation. At one end of the test piece in the longitudinal direction, about 5 mm was peeled from the release liner end portion and folded back at an angle of 180°, and the aid paper (width 5 mm) was adhered to extend the length. Using a tensile tester, one end of the aid paper was nipped by the upper clasp, the release liner and the plate for backing e.g., Bakelite (trade name) plate) were nipped by the lower clasp, the adhesive sheet was continuously peeled off in the 180 degree direction at 300 mm/min under an atmosphere of 23°C$\pm$2°C, 50$\pm$10%RH, and the peel force was measured. The results are shown in Table 1.

Experimental Example 2

<Evaluation of taking-out performance of adhesive patch from package>

The patch preparations of Examples 1 - 8 and Comparative Examples 1, 2 were placed in packages formed from a packaging material (outer size: 100 mm x 100 mm, inner size: 90 mm x 90 mm) having an outer layer made of a 12 $\mu$m-thick PET film and an inner layer made of a 30 $\mu$m-thick polyacrylonitrile resin film, and the packages were tightly sealed. Then, as shown in Fig. 3, two sides of the adhesive patch package were opened with scissors or along V-shaped notches. The adhesive patch was taken out by holding a corner thereof, and the taking-out performance of the adhesive patch from the package was evaluated based on the following evaluation criteria and using evaluation points 1 - 5. The results are shown in Table 1.

<Evaluation criteria>

5: end portion of patch preparation is not lifted and patch preparation can be taken out extremely easily
4: end portion of patch preparation is partly lifted but the adhesive surface does not attach and patch preparation can be taken out easily
3: end portion of patch preparation is partly lifted, adhesive surface slightly attaches to package inside, but patch preparation can be taken out easily
2: end portion of patch preparation is lifted, adhesive surface attaches to package inside, and patch preparation is difficult to take out
1: most of patch preparation is lifted, the adhesive surface attaches to package inside, and patch preparation is extremely difficult to take out

Experimental Example 3

<Evaluation of easy releaseability of a release liner>

Four panelists detached a release liner from the cutting part of a patch preparation, and evaluated the easy releaseability thereof by sensory evaluation. The evaluation results were summarized by the following criteria. The results are shown in Table 1.

<Summary criteria of evaluation>

5: all 4 panelists felt easy detachability
4: 3 out of 4 panelists felt easy detachability
3: 2 out of 4 panelists felt easy detachability
2: 1 out of 4 panelists felt easy detachability
1: all 4 panelists felt difficulty in detachment

Table 1

| | peel force (mN/2 mm) in central part | treatment of peripheral part | peel force (mN/2 mm) in treated area of peripheral part | taking-out performance from package | detach-easiness of release liner |
|---|---|---|---|---|---|
| Example 1 | 11 | complete removal of release agent | 493 | 5 | 4 |
| Example 2 | 14 | removal of release agent of one side | 474 | 4 | 5 |
| Example 3 | 13 | complete removal of release agent of all corner areas | 498 | 5 | 5 |
| Example 4 | 20 | complete removal of release agent | 40 | 5 | 5 |
| Example 5 | 19 | removal of release agent of countering two sides | 43 | 5 | 5 |
| Example 6 | 17 | removal of release agent of two corner areas located on diagonal line | 42 | 4 | 5 |
| Example 7 | 2 | complete removal of release agent | 7 | 3 | 5 |
| Example 8 | 8 | removal of release agent of countering two sides | 29 | 4 | 5 |
| Comparative Example 1 | 829 | none | 829 | 5 | 1 |
| Comparative Example 2 | 2 | none | 2 | 1 | 5 |

This application is based on a patent application No. 2011-283713 filed in Japan, the contents of which are incorporated in full herein.

**Claims**

1. A patch preparation having a central part and a peripheral part surrounding the central part, the patch preparation comprising: a patch preparation main body having a support and an adhesive layer which contains a drug and is formed on the support; and a release liner laminated on the adhesive layer, wherein the peripheral part has a predetermined area, and a peel force between the adhesive layer and the release liner in the predetermined area is greater than a peel force between the adhesive layer and a release liner in the central part.

2. The patch preparation according to claim 1, wherein the patch preparation has a substantially rectangular or a substantially square planar shape.

3. The patch preparation according to claim 2, wherein the peripheral part has four corner areas, and the predetermined area is at least one of the four corner areas.

4. The patch preparation according to claim 2, wherein the peripheral part has four corner areas and the predetermined area is one pair of two countering corner areas out of the four corner areas.

5. The patch preparation according to claim 2, wherein the peripheral part has four corner areas, and the predetermined area is the four corner areas.

6. The patch preparation according to claim 2, wherein the peripheral part has four sides, and the predetermined area is an area along one side out of the four sides.

7. The patch preparation according to claim 2, wherein the peripheral part has four sides, and the predetermined area is two areas along each one pair of the two countering sides out of the four sides.

8. The patch preparation according to claim 1 or 2, wherein the predetermined area is an entire area of the peripheral area.

9. The patch preparation according to any one of claims 1 to 8, wherein the peel force of the predetermined area is 5 to 500 mN/2mm.

10. The preparation according to any one of claims 1 to 9, wherein the peel force of the predetermined area is greater than the peel force between the adhesive layer and a release liner in the central part by not less than 3 mN/2mm.

11. The patch preparation according to any one of claims 1 to 10, wherein the peripheral area has a width of 0.5 to 10 mm.

12. The patch preparation according to any one of claims 1 to 11, wherein the adhesive layer has a thickness of 10 to 500 $\mu$m.

13. The patch preparation according to any one of claims 1 to 12, wherein the adhesive layer comprises an organic liquid component.

14. The patch preparation according to any one of claims 1 to 13, wherein the release liner covers the entire exposed surface of the adhesive layer of the patch preparation main body;
wherein optionally an end portion of the release liner protrudes from the end portion of the patch preparation main body.

15. The patch preparation according to any one of claims 1 to 14, wherein the release liner has a continuous cutting line.

## FIG. 1

(A)

5 A  5 B  5

5 A

4

3

W1

(B)

5 B  5 A  5 B  5

4

2

3

1

W1

## FIG. 2

10

5

6

4

2

3

1

## FIG. 3

## FIG. 4

(A)

(D)

(B)

(E)

(C)

Europäisches
Patentamt

European
Patent Office

Office européen
des brevets

**EUROPEAN SEARCH REPORT**

Application Number

EP 12 19 9165

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 482 491 B1 (SAMUELSEN PETER BOMAN [DK] ET AL) 19 November 2002 (2002-11-19) * the whole document * | 1-15 | INV. A61K9/70 A61F13/02 |
| A | US 2010/056972 A1 (HARIMA JUN [JP] ET AL) 4 March 2010 (2010-03-04) * paragraph [0010] - paragraph [0030] * * paragraph [0104] - paragraph [0115] * * figure 4 * | 1-15 | |
| A | US 2010/055162 A1 (HARIMA JUN [JP] ET AL) 4 March 2010 (2010-03-04) * figures 1a, 1b * * paragraph [0010] - paragraph [0014] * | 1-15 | |

TECHNICAL FIELDS
SEARCHED (IPC)

A61F
A61K

The present search report has been drawn up for all claims

| Place of search | Date of completion of the search | Examiner |
|---|---|---|
| The Hague | 25 April 2013 | González Ferreiro, M |

EPO FORM 1503 03.82 (P04C01)

## ANNEX TO THE EUROPEAN SEARCH REPORT
## ON EUROPEAN PATENT APPLICATION NO.

EP 12 19 9165

This annex lists the patent family members relating to the patent documents cited in the above-mentioned European search report.
The members are as contained in the European Patent Office EDP file on
The European Patent Office is in no way liable for these particulars which are merely given for the purpose of information.

25-04-2013

| Patent document cited in search report | | Publication date | Patent family member(s) | | Publication date |
|---|---|---|---|---|---|
| US 6482491 | B1 | 19-11-2002 | AT | 263226 T | 15-04-2004 |
| | | | AU | 2264799 A | 16-08-1999 |
| | | | DE | 69916025 D1 | 06-05-2004 |
| | | | DE | 69916025 T2 | 28-10-2004 |
| | | | EP | 1051451 A1 | 15-11-2000 |
| | | | US | 6482491 B1 | 19-11-2002 |
| | | | WO | 9938929 A1 | 05-08-1999 |
| US 2010056972 | A1 | 04-03-2010 | AU | 2009212842 A1 | 18-03-2010 |
| | | | BR | PI0902665 A2 | 25-05-2010 |
| | | | CA | 2676757 A1 | 28-02-2010 |
| | | | CN | 101658512 A | 03-03-2010 |
| | | | EP | 2158884 A2 | 03-03-2010 |
| | | | JP | 2010053064 A | 11-03-2010 |
| | | | KR | 20100027052 A | 10-03-2010 |
| | | | NZ | 579246 A | 24-12-2010 |
| | | | RU | 2009132397 A | 10-03-2011 |
| | | | US | 2010056972 A1 | 04-03-2010 |
| US 2010055162 | A1 | 04-03-2010 | AT | 521315 T | 15-09-2011 |
| | | | AU | 2009212843 A1 | 18-03-2010 |
| | | | BR | PI0903047 A2 | 25-05-2010 |
| | | | CA | 2676756 A1 | 28-02-2010 |
| | | | CN | 101658511 A | 03-03-2010 |
| | | | EP | 2158885 A1 | 03-03-2010 |
| | | | ES | 2369053 T3 | 24-11-2011 |
| | | | JP | 2010053065 A | 11-03-2010 |
| | | | KR | 20100027049 A | 10-03-2010 |
| | | | NZ | 579247 A | 24-12-2009 |
| | | | RU | 2009132396 A | 10-03-2011 |
| | | | US | 2010055162 A1 | 04-03-2010 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10310108 A
- JP 6199659 A

- JP 2011283713 A